# FASCICULE DE BREVET EUROPEEN

(11) **EP 4 373 458 B1**
(45) Date de publication et mention de la délivrance du brevet: **11.06.2025**
(21) Numéro de dépôt: 22754124.0
(22) Date de dépôt: 20.07.2022
(51) Int. Cl.: A61K 8/06, A61K 8/39, A61K 8/49, A61K 8/60, A61K 8/73, A61Q 19/00, A61Q 19/08

(54) **COMPOSITION COSMETIQUE SOUS LA FORME D'EMULSION HUILE-DANS-EAU A FORTE TENEUR EN INGREDIENTS D'ORIGINE NATURELLE**
KOSMETISCHE ZUSAMMENSETZUNG IN FORM EINER ÖL-IN-WASSER-EMULSION MIT EINEM HOHEN GEHALT AN NATÜRLICH VORKOMMENDEN INHALTSSTOFFEN
COSMETIC COMPOSITION IN THE FORM OF AN OIL-IN-WATER EMULSION WITH A HIGH CONTENT OF NATURALLY OCCURRING INGREDIENTS

(30) Priorité: 21.07.2021 FR 2107889
(43) Date de publication de la demande: 29.05.2024
(73) Titulaire: Laboratoires Clarins, 75017 Paris (FR)
(72) Inventeur: FOUCAULT, Sophie, 78570 ANDRESY (FR); BERNADOU, Caroline, 95000 CERGY (FR)
(74) Mandataire: Novagraaf Technologies
(86) Numéro de dépôt international: PCT/FR2022/051442
(87) Numéro de publication internationale: WO 2023/002123

(56) Documents cités:
- EP-A1- 1 679 063
- EP-A1- 2 218 440
- EP-B1- 3 267 964
- WO-A1-2020/043363

## Description

### Domaine technique

La présente invention se rapporte au domaine des compositions cosmétiques de type émulsion huile-dans-eau (H/E) à forte teneur en ingrédients d'origine naturelle, dédiées notamment au soin de la peau comme par exemple des compositions pour hydrater, traiter et/ou atténuer les signes de vieillissement cutané.

La présente invention concerne également un procédé de soin cosmétique non thérapeutique comprenant l'application sur la peau d'une composition cosmétique selon l'invention et l'utilisation de cette composition cosmétique pour le soin de la peau.

### Arrière-plan technologique

Les compositions cosmétiques pour le soin de la peau ont évolué depuis de nombreuses années et demeurent une demande très forte des consommateurs, par exemple pour hydrater, traiter, améliorer l'apparence de la peau et/ou atténuer les signes de vieillissement cutané.

Différentes formes galéniques existent dans le commerce afin de fournir différentes textures et/ou un certain confort lors de l'utilisation par le consommateur. Elles peuvent être par exemple sous la forme d'huiles, de crèmes, de baumes, de masques, de gels, de cires ou de sérum en fonction de la texture, la fluidité ou la viscosité souhaitée pour une application donnée. Les compositions sous forme d'émulsion huile-dans-eau (ou émulsions « directes ») sont plus particulièrement appréciées dans le domaine cosmétique car elles procurent une meilleure sensorialité que les émulsions eau-dans-huile, comme par exemple une sensation plus légère, moins grasse, moins collante, plus fraîche à l'application. Ces émulsions sont stabilisées à l'aide d'émulsionnants ou de tensioactifs et certaines textures épaisses, comme par exemple les baumes, nécessitent l'ajout de gélifiants ou épaississants de phase aqueuse.

Aujourd'hui plus que jamais, des compositions avec des ingrédients plus respectueux de l'environnement sont au cœur des préoccupations des consommateurs qui sont en outre de plus en plus avertis et exigeants. La demande est donc forte en produits naturels et/ou éco-conçus, c'est-à-dire dont la conception et le développement tiennent compte des impacts environnementaux (matières premières et leurs procédés de fabrication, gestion durable des ingrédients naturels, logistique et distribution du produit, packaging, biodégradabilité, etc). Plus particulièrement, les consommateurs sont en recherche de produits cosmétiques ayant un taux élevé d'ingrédients naturels et/ou d'origine naturelle.

Les règlementations ont également évolué dans ce sens afin de mieux quantifier le taux d'ingrédients d'origine naturelle dans un produit. En particulier, pour déterminer le degré de naturalité de leurs ingrédients développés et fabriqués, les industriels utilisent la norme NF ISO 16128 qui définit différentes catégories d'ingrédients d'origine biologique et/ou naturelle et donne des directives pour le calcul du pourcentage d'ingrédients d'origine naturelle de leur matière première finie et l'attribution d'un indice de naturalité aux ingrédients cosmétiques.

Par ailleurs, comme toute autre composition cosmétique conventionnelle, les compositions cosmétiques éco-conçues et/ou naturelles doivent également satisfaire à des critères de performance et d'usage (homogénéité, stabilité, applicabilité, innocuité, conservation).

Dans le même temps, les consommateurs restent très exigeants sur les qualités sensorielles et la performance des produits. Ils continuent notamment de rechercher des textures légères, agréables au toucher et avec de bonnes propriétés sensorielles comme par exemple un toucher doux, non gras, non collant, une application facile sur la peau.

Or l'utilisation d'ingrédients d'origine naturelle a un impact important sur la sensorialité d'un produit cosmétique mais aussi sur sa stabilité et son homogénéité. Par exemple, les formules ayant un taux important d'ingrédients naturels et/ou d'origine naturelle présentent des galéniques souvent perçues comme « pâteuses » et pouvant générer un frein à l'application.

Certains émulsionnants naturels, comme les alcools gras, présentent l'inconvénient de produire un effet de savonnage à l'application (ou effet blanchissant à l'application). Pour pallier ce problème, il est connu d'introduire des silicones dans les formules pour supprimer l'effet savon à l'application de l'émulsion. Mais ces matières siliconées ne sont pas d'origine naturelle et ont un impact sur l'environnement.

Quant aux gélifiants naturels, ils ont un pouvoir gélifiant et/ou stabilisant d'émulsion peu efficace à faible pourcentage, contrairement aux gélifiants synthétiques. L'utilisation de pourcentages plus élevés de gélifiants naturels permet de favoriser la stabilité mais a un impact négatif sur la sensorialité, avec en général un effet collant, glaireux et/ou filant. En outre, les gélifiants naturels présentent l'inconvénient de conduire à des produits qui peluchent à l'application.

Par ailleurs, en fonction de la galénique souhaitée, des ingrédients lipophiles (d'origine naturelle) sont également nécessaires pour apporter de la consistance au produit, comme par exemple des beurres, des alcools gras ou des glycérides d'acide gras. Or, en l'absence de gélifiant synthétique, ces ingrédients ont tendance à cristalliser de manière anarchique et créent des grains visibles dans la texture. Un problème supplémentaire est donc la maîtrise de ces phénomènes de cristallisation.

Ainsi, les difficultés techniques sont nombreuses pour formuler des émulsions cosmétiques comprenant à la fois un taux élevé d'ingrédients d'origine naturelle et ayant des performances satisfaisantes en termes de stabilité et/ou de sensorialité. Des compositions plus naturelles sont connues. On peut citer par exemple les compositions décrites dans le document WO2020/254420. Mais ces compositions contournent les difficultés en utilisant un gélifiant synthétique pour obtenir une stabilité, une consistance et une sensorialité suffisantes. Or la pollution liée aux polymères synthétiques, qui s'accumulent et persistent notamment dans les eaux et océans, reste un défi de taille à notre époque. EP2218440 décrit par ailleurs un système solubilisant des composés lipophiles compatible avec la formulation de produits cosmétiques naturels et/ou " certifiés d'origine biologiques, comprenant l'association d'un ester de sucrose et d'acide gras et d'un ester de polyglycerol et d'acides gras.

Il existe donc toujours un besoin de formuler des compositions cosmétiques présentant un taux élevé d'ingrédients naturels et/ou d'origine naturelle, et en particulier exemptes de polymères synthétiques.

En particulier, il existe un besoin de disposer de compositions cosmétiques, notamment pour le soin de la peau, qui présentent un taux de naturalité élevé, comprenant par exemple au moins 95% d'ingrédients d'origine naturelle, qui est un critère recherché par de plus en plus de consommateurs.

Il existe également un besoin de formuler des émulsions cosmétiques de consistance suffisante, comprenant à la fois un taux élevé d'ingrédients d'origine naturelle (taux de naturalité global du produit cosmétique) et ayant des performances satisfaisantes en termes de sensorialité (facilité d'application sur la peau, sans sensation de freinage, non collante, sans peluchage, sans effet savon, sans sensation de gras ou de film épais sur la peau, agréables et douces au toucher, etc.).

En parallèle, il existe un besoin de disposer de compositions cosmétiques présentant une bonne stabilité et homogénéité dans le temps (sans phénomènes de relargage, de cristallisation ou de casse de l'émulsion), en particulier qui soient stables pendant toute la période de stockage du produit jusqu'à plusieurs mois après son ouverture par l'utilisateur final.

La demanderesse a observé de façon inattendue et surprenante que l'ensemble de ces problèmes peuvent être résolus par la composition selon la présente invention.

### Résumé de l'invention

Selon un aspect, la présente invention a pour objet une composition cosmétique sous forme d'émulsion huile-dans-eau comprenant au moins une phase grasse dispersée dans une phase aqueuse et comprenant :
a) au moins un ester de sucre et d'acide gras ;
b) au moins un ester de sorbitan et d'acide gras ;
c) au moins un ester de polyglycérol et d'acide gras ;
d) au moins une gomme xanthane ; et
e) au moins une gomme gellane.

En effet, après de nombreuses recherches, les inventeurs ont mis en évidence qu'il est possible de fournir une composition sous forme d'émulsion huile-dans-eau, présentant une consistance suffisante, à forte teneur en ingrédients d'origine naturelle et qui possède également les propriétés sensorielles et de stabilité physico-chimique d'une émulsion composée d'ingrédients d'origine pétrochimique. En d'autres termes, les inventeurs ont mis au point une composition permettant de cumuler les avantages des émulsions à fort taux de naturalité avec un faible impact environnemental (écotoxicité, biodégradabilité) avec la qualité sensorielle et les performances de stabilité des émulsions composées d'ingrédients pétrochimiques.

En particulier, les compositions selon l'invention réunissent, de façon surprenante, l'ensemble des propriétés recherchées telles que l'aspect attractif (lisse et non grumeleux), une consistance suffisante et en même temps une texture légère, non « pâteuse », facile à appliquer sur la peau (non freinante), non collante, avec un effet « savon » peu présent à l'application, ainsi qu'un effet "peluchage" faible et acceptable. L'association selon l'invention permet notamment d'obtenir une viscosité suffisante pour conditionner la composition en pot, par exemple sous forme de texture crème, voire de texture baume.

En outre, l'association selon l'invention permet l'obtention de compositions ayant une très bonne stabilité dans le temps comme démontré dans les exemples. Avantageusement, la composition selon l'invention contient au moins 95% d'ingrédients d'origine naturelle, de préférence au moins 99% d'ingrédients d'origine naturelle. Le pourcentage d'ingrédient d'origine naturelle est ici calculé selon les principes de la norme NF ISO 16128. De façon surprenante, la combinaison particulière d'ingrédients mise en évidence par les inventeurs permet d'obtenir des compositions ayant un taux de naturalité très élevé, tout en préservant les propriétés sensorielles et de stabilité des compositions obtenues avec des ingrédients d'origine pétrochimique.

Avantageusement, la composition selon l'invention est exempte de composés siliconés et/ou de gélifiants synthétiques ou d'origine pétrochimique. En effet, l'invention présente l'avantage de fournir des émulsions huile-dans-eau stables et sensorielles sans nécessiter l'ajout de ce type de composés.

La composition selon l'invention est adaptée à une application topique sur la peau, notamment saine. Elle peut être utilisée pour le soin cosmétique et/ou le maquillage, notamment de la peau. Par exemple, elle peut être utilisée comme produit de soin cosmétique de la peau, par exemple pour hydrater, protéger, traiter la peau et/ou atténuer les signes de vieillissement cutané. Elle peut être par exemple utilisée pour le visage, pour le contour des yeux, pour les mains ou pour le corps.

Ainsi, l'invention a également pour objet l'utilisation de la composition cosmétique telle que définie ci-dessus comme produit de soin cosmétique, de préférence pour hydrater, protéger, traiter la peau et/ou atténuer les signes de vieillissement cutané.

### Description détaillée

Les termes généraux utilisés dans la présente sont définis ci-dessous.

L'expression "au moins un(e)" est équivalente à l'expression "un(e) ou plusieurs".

L'expression "comprenant" englobe l'expression "consistant en".

L'expression "de ... à ..." doit se comprendre bornes incluses.

Par "composition cosmétique", on entend au sens de la présente invention une composition adaptée à une application topique externe. Elle comprend un support cosmétiquement acceptable, c'est à dire un support compatible avec la peau (corps, visage, contour des yeux, paupières). La composition cosmétique peut être un produit de soin et/ou de maquillage, de préférence un produit de soin de la peau.

Par "émulsion huile-dans-eau" (notée H/E), on entend une composition constituée d'une phase aqueuse continue dans laquelle est dispersée une phase discontinue grasse, notamment sous forme de gouttelettes, de manière à observer un mélange macroscopiquement homogène à l'œil nu.

Par "émulsionnant", on entend au sens de la présente invention tout composé ou mélange de composés susceptible d'augmenter la stabilité cinétique d'une émulsion. Au sens de la présente invention, ce terme désigne des composés amphiphiles, c'est-à-dire qui possèdent à la fois une partie hydrophile (polaire) et une partie lipophile (apolaire).

Par ingrédient "naturel", on entend au sens de la présente invention un ingrédient (ou une substance) présent dans la nature (obtenu à partir de végétaux, d'animaux, de micro-organismes ou de minéraux), non transformé ou obtenu à partir de processus physique uniquement. Par exemple l'ingrédient peut être extrait et/ou traité par des moyens mécaniques ou gravitationnels, par dissolution dans l'eau, par macération, par extraction par l'eau, par distillation, par broyage, par pression, par flottation, par sédimentation, par filtration, etc. L'indice de naturalité d'un ingrédient naturel, déterminé selon la norme NF ISO n° 16128 (ou "indice d'origine naturelle"), est de 1 (ingrédient 100% d'origine naturelle). A titre d'ingrédient naturel, on peut par exemple citer l'eau, des extraits de plantes dont les solvants utilisés sont naturels (eau ou autres solvants naturels), des parties de plantes ou de graines, des huiles végétales, des ingrédients minéraux, etc.

Par ingrédient "dérivé de naturel", on entend au sens de la présente invention un ingrédient naturel extrait et/ou traité chimiquement, ayant subi des transformations chimiques de faible ampleur et en nombre limité. Ces transformations et processus chimiques peuvent se faire selon les principes de la chimie verte (par opposition aux procédés pétrochimiques faisant intervenir des dérivés de pétrole) et sont par exemple listés dans la norme NF ISO n°16128. L'indice de naturalité d'un ingrédient « dérivé de naturel », calculé selon la norme NF ISO n°16128, est supérieur à 0,5 (c'est-à-dire qu'il contient une proportion de plus de 50% de la masse moléculaire issue de la nature).

Le terme ingrédient "d'origine naturelle" regroupe ici les ingrédients "naturels" et les ingrédients "dérivés de naturel" tel que définis précédemment. L'indice de naturalité d'un ingrédient d'origine naturelle, calculé selon la norme NF ISO n°16128, est supérieur à 0,5 et inférieur ou égale à 1. Par opposition, les ingrédients synthétiques ou issus majoritairement de combustibles fossiles ont un indice de naturalité de 0. Le nom "INCI" désigne le nom d'un ingrédient cosmétique selon la nomenclature internationale (International Nomenclature of Cosmetic Ingredients).

Par "exempte", on entend au sens de la présente invention une quantité massique d'ingrédient (ou de substance) inférieure ou égale à 0,1% par rapport à la masse totale de la composition, de préférence inférieure ou égale à 0,05%.

Sauf mention contraire, on entend par "acide gras" au sens de la présente invention un acide carboxylique comprenant 8 à 24 atomes de carbone. Les acides gras sont de préférence monocarboxyliques. Ils peuvent être saturés ou insaturés, linéaires ou ramifiés.

### Ester de sucre et d'acide gras :

La composition selon l'invention comprend au moins un ester de sucre et d'acide gras (également appelé "sucroester").

Ce type de composant est connu comme tensioactif non ionique stabilisateur d'émulsion.

Il s'agit d'un ester issu de la réaction d'un (ou plusieurs) acide(s) gras et d'un sucre, de préférence le saccharose. Il peut donc s'agir de mono ou de polyesters d'acides gras et de saccharose. L'ester de sucre et d'acide gras peut éventuellement se présenter sous forme d'un mélange de mono et de polyesters d'un même acide gras. Les esters de sucre et d'acides gras ont de préférence un faible degré d'estérification comme par exemple des monoesters, diesters et triesters de sucre et d'acide gras. De préférence, l'ester de sucre et d'acide gras est choisi parmi les mono et diesters d'acides gras de saccharose.

Les acides gras sont des acides carboxyliques, de préférence monocarboxyliques, saturés ou insaturés, linéaires ou ramifiés. De préférence, les acides gras sont des acides monocarboxyliques, linéaires et saturés.

De préférence, les acides gras de l'ester de sucre comprennent 10 à 24 atomes de carbone, de préférence de 12 à 20 atomes de carbone.

A titre d'exemples non limitatifs d'acides gras, on peut citer l'acide laurique, l'acide palmitique, l'acide stéarique, l'acide oléique.

Des exemples non limitatifs d'esters d'acide gras de saccharose (ou sucroesters) utilisables dans la composition selon l'invention comprennent le sucrose cocoate, le sucrose stearate, le sucrose laurate, le sucrose palmitate, le sucrose myristate, le sucrose polystéarate (également appelé polystéarate de saccharose) et leur mélange. La composition selon la présente invention peut éventuellement comprendre un mélange de deux ou plusieurs sucroesters de différents acides gras.

A titre d'exemples non limitatifs d'ester de sucre et d'acide gras ou de mélanges comprenant au moins un ester de sucre et d'acide gras, on peut citer les produits commercialisés sous les dénominations :
- Arlacel 2121 (nom INCI : sorbitan stearate & sucrose cocoate) de la société CRODA,
- Tegosoft PSE 141G (nom INCI : sucrose stearate) de la société EVONIK GOLDSCHMIDT,
- Surfhope SE COSME C-1816 fabriqué par la société MITSUBISHI KAGAKU FOOD (nom INCI : sucrose stearate),
- Surfhope SE COSME C-1416 fabriqué par la société MITSUBISHI KAGAKU FOOD (nom INCI : sucrose myristate),
- Surfhope SE COSME C-1216 fabriqué par la société MITSUBISHI KAGAKU FOOD (nom INCI : sucrose laurate),
- Surfhope SE COSME C-1215L fabriqué par la société MITSUBISHI KAGAKU FOOD (nom INCI : sucrose laurate),
- Surfhope SE COSME C-1616 fabriqué par la société MITSUBISHI KAGAKU FOOD, (nom INCI sucrose palmitate).

Avantageusement, l'esters de sucre et d'acide gras est le sucrose cocoate.

La teneur massique en ester de sucre et d'acide gras dans la composition selon la présente invention peut être d'au moins 0,2%, de préférence d'au moins 0,3% par rapport à la masse totale de la composition.

D'autre part, la teneur massique en ester de sucre et d'acide gras dans la composition selon la présente invention peut être d'au plus 0,8%, de préférence au plus 0,55% par rapport à la masse totale de la composition.

De préférence, la teneur massique en ester de sucre et d'acide gras dans la composition selon la présente invention est de 0,2% à 0,8%, de préférence de 0,2% à 0,6%, de préférence de 0,3% à 0,55%, par rapport à la masse totale de la composition. Ces proportions préférées permettent de combiner une meilleure stabilité et sensorialité de la composition.

### Ester d'acide gras du sorbitan :

La composition selon l'invention comprend au moins un ester de sorbitan et d'acide gras.

Les esters de sorbitan et d'acide gras sont connus pour être des tensioactifs non ioniques stabilisateurs d'émulsion. Il peut s'agir de mono ou de polyesters d'acides gras du sorbitan. En raison de la manière dont ils sont généralement fabriqués, les esters de sorbitan peuvent comprendre des mélanges de mono, di, tri, ou polyesters d'un même acide gras. De préférence, l'ester de sorbitan et d'acide gras est choisi parmi les mono et diesters d'acides gras du sorbitan.

Les acides gras sont des acides carboxyliques, de préférence monocarboxyliques, saturés ou insaturés, linéaires ou ramifiés. De préférence, les acides gras sont des acides monocarboxyliques, linéaires et saturés.

De préférence, les acides gras de l'ester de sorbitan comprennent 10 à 24 atomes de carbone, de préférence de 12 à 20 atomes de carbone.

Par exemple, parmi les acides gras on peut citer l'acide laurique, l'acide palmitique, l'acide stéarique, l'acide oléique.

Des exemples non limitatifs d'esters d'acide gras de sorbitan utilisables dans la composition selon l'invention comprennent le monolaurate de sorbitan, l'oléate de sorbitan, le stéarate de sorbitan, l'isostéarate de sorbitan, le palmitate de sorbitan, le myristate de sorbitan et leur mélange.

A titre d'exemples non limitatifs d'ester de sorbitan et d'acide gras ou de mélanges comprenant au moins un ester de sorbitan et d'acide gras, on peut citer les produits commercialisés sous les dénominations :
- Arlacel 2121 (nom INCI : sorbitan stearate & sucrose cocoate) de la société CRODA,
- Span 60 (nom INCI : sorbitan stearate) de la société CRODA,
- Span 120 (nom INCI : sorbitan isostearate) de la société CRODA,
- Span 65 (nom INCI : sorbitan tristearate) de la société CRODA,
- Span 80 (nom INCI : sorbitan oleate) de la société CRODA.

Avantageusement, l'ester de sorbitan et d'acide gras est le sorbitan stéarate.

La teneur massique en esters d'acide gras du sorbitan dans la composition selon la présente invention peut être d'au moins 1,8%, de préférence d'au moins 2,7% par rapport à la masse totale de la composition.

D'autre part, la teneur massique en esters d'acide gras du sorbitan dans la composition selon la présente invention peut être d'au plus 5,4%, de préférence au plus 4,5% par rapport à la masse totale de la composition.

De préférence, la teneur massique en esters d'acide gras du sorbitan dans la composition selon la présente invention est de 1,8% à 5,4%, de préférence de 2,7% à 4,5%, par rapport à la masse totale de la composition. Cela permet de combiner une meilleure stabilité et sensorialité de la composition.

### Ester d'acide gras de polyglycérol :

La composition selon l'invention comprend au moins un ester de polyglycérol et d'acide gras.

Par ester de polyglycérol et d'acide gras, on désigne au sens de l'invention un ester résultant de la réaction d'un polyglycérol avec au moins un acide gras.

Un polyglycérol désigne ici un homopolymère de glycérol comprenant au moins deux unités glycérol, de préférence 3 à 10 unités glycérol, de préférence 4 à 8 unités glycérol. Plus préférentiellement, le polyglycérol comprend 6 unités glycérol.

Les esters de polyglycérol et d'acides gras peuvent être choisis parmi les mono-, di-, tri-, tétra-esters, les polyesters et leurs mélanges. On utilise de préférence des esters ou mélanges d'esters à faible degré d'estérification comme par exemple des monoesters, diesters ou triesters de polyglycérol et d'acide gras.

Les acides gras sont définis précédemment.

De préférence, les acides gras de l'ester de polyglycérol comprennent 10 à 22 atomes de carbone, de préférence de 14 à 22 atomes de carbonne.

Ils peuvent par exemple être choisis parmi l'acide oléique, l'acide stéarique, l'acide isostéarique, l'acide laurique, l'acide palmitique, l'acide myristique, l'acide linoléique, l'acide caprique, l'acide caprylique, ou leurs mélanges.

Des exemples non limitatifs d'esters d'acide gras de polyglycérols utilisables dans la composition selon l'invention comprennent le polyglyceryl-4 caprate (ester issu de la réaction de polyglycérol comprenant 4 unités glycérol et d'acide caprique), le polyglyceryl-6 distearate (diester issu de la réaction de polyglycérol comprenant 6 unités glycérol et d'acide stéarique), le polyglyceryl-6 stearate, polyglyceryl-6 isostearate, le polyglyceryl-6 behenate, le polyglyceryl-6 caprylate, le polyglyceryl-6 ricinoleate, le polyglyceryl-6 polyricinoleate, le polyglyceryl-10 myristate, le polyglyceryl-10 laurate et leur mélange.

A titre d'exemples non limitatifs, on peut citer les produits commercialisés sous les dénominations :
- TEGOCARE PBS 6 (nom INCI : Polyglyceryl-6 Stearate & Polyglyceryl-6 Behenate ; no. CAS 9009-32-9 et 64366-79-6) de la société EVONIK NUTRITION & CARE GMBH,
- TEGO SOLVE 90 (nom INCI : Polyglyceryl-6 Caprylate & Polyglyceryl-4 Caprate & Aqua/Water/Eau) de la société EVONIK NUTRITION & CARE GMBH,
- TEGO SOLVE 61 (nom INCI : Polyglyceryl-6 Caprylate & Polyglyceryl-3 Cocoate & Polyglyceryl-4 Caprate & Polyglyceryl-6 Ricinoleate & Aqua/Water/Eau) de la société EVONIK NUTRITION & CARE GMBH,
- EMULIUM ILLUSTRO (nom INCI : Polyglyceryl-6 Polyhydroxystearate & Polyglyceryl-6 Polyricinoleate) commercialisé par la société GATTEFOSSE,
- DERMOFEEL G10 LW 70 MB (nom INCI : Polyglyceryl-10 Laurate & Aqua & Citric Acid) de la société EVONIK NUTRITION & CARE GMBH.

De préférence, le au moins un ester de polyglycérol et d'acide gras est choisi parmi le polyglyceryl-6 stearate, le polyglyceryl-6 behenate et leur mélange.

De préférence, la composition selon l'invention comprend au moins deux esters de polyglycérol et d'acide gras. Avantageusement, la composition selon l'invention comprend le polyglyceryl-6 stearate et le polyglyceryl-6 behenate.

La teneur massique en ester d'acide gras de polyglycérol dans la composition selon la présente invention peut être d'au moins 0,5%, de préférence d'au moins 1%, et plus préférablement au moins 2%, par rapport à la masse totale de la composition.

D'autre part, la teneur massique en esters d'acide gras de polyglycérol dans la composition selon la présente invention peut être d'au plus 6%, de préférence au plus 3%, par rapport à la masse totale de la composition.

De préférence, la teneur massique en esters d'acide gras de polyglycérol dans la composition selon la présente invention est de 0,5% à 6%, de préférence de 1% à 6%, de préférence de 2% à 3%, par rapport à la masse totale de la composition. Cela permet de combiner une meilleure stabilité et sensorialité de la composition.

### Epaississants aqueux :

La composition selon l'invention comprend au moins deux épaississants aqueux choisis parmi une gomme de xanthane et une gomme de gellane.

Par "épaississant" (également appelé ici gélifiant), on entend au sens de la présente invention un polymère qui, de par sa présence, permet d'augmenter la viscosité de la composition dans laquelle il est introduit.

Par "épaississant aqueux", on entend au sens de la présente invention un polymère hydrosoluble ou hydrodispersible ou gonflable dans l'eau.

Après de nombreuses recherches, les inventeurs ont mis en évidence que l'association des deux gélifiants particuliers que sont la gomme xanthane et la gomme gellane permet d'obtenir à la fois une texture crème de viscosité suffisante et une meilleure stabilisation de l'émulsion (stabilisation des gouttelettes d'huile de la phase grasse). En outre, associés avec le mélange d'émulsionnants, la combinaison particulière d'ingrédients selon l'invention permet de limiter de façon surprenante les phénomènes de cristallisation d'agents de consistance lipophiles d'origine naturelle (absence de grains ou d'aspect grumeleux).

Avantageusement, la composition selon l'invention est exempte d'épaississant synthétique aqueux. A titre d'exemples non limitatifs d'épaississants synthétiques aqueux, on peut citer les polyacrylamides, les polymères et copolymères d'acide acrylamido 2-méthylpropane sulfonique (comme les copolymères à motif acryloyldiméthyltaurate), les polymères carboxyvinyliques (comme les carbomères, ou polymères synthétiques hydrophiles d'acide acrylique) ou les polyuréthanes.

La composition cosmétique selon la présente invention comprend de la gomme de xanthane.

La gomme de xanthane est un polysaccharide naturel ramifié. Ce polyoside est constitué d'un squelette cellulosique (chaîne principale de résidus D-glucose) substitué, sur des résidus de glucose alternés, par une chaîne latérale trisaccharidique contenant un résidu mannose, généralement acétylé, un résidu acide glucuronique et un résidu mannose terminal. En outre, un résidu d'acide pyruvique est porté par certains résidus mannose terminal (généralement par 30% à 50% des résidus mannose terminal). Les résidus acides glucuroniques et acides pyruviques sont ionisables et donc responsables de la nature anionique du xanthane. Ils peuvent être neutralisés dans les produits commerciaux avec des cations tels que Na⁺, K⁺ ou Ca²⁺.

Les gommes xanthane sont produites à l'échelle industrielle, en tant qu'exopolysaccharide, par la fermentation de la bactérie *Xanthomonas campestris.* La proportion de résidus pyruvate et groupements acétate peut varier selon la souche de bactérie, le procédé de fermentation, les conditions après fermentation et les étapes de purification.

A titre d'exemples non limitatifs de gommes xanthanes commercialement disponibles, on peut citer les composés de no. CAS 11138-66-2 ou les produits commercialisés sous les dénominations Rhodicare XC de la société DANISCO, Keltrol CG-SFT ou Keltrol CG-V de la société CP KELCO, les produits de la gamme Satiaxane (tels que Satiaxane VPC 930) de la société CARGILL.

La teneur massique en gomme xanthane dans la composition selon la présente invention peut être d'au moins 0,2%, de préférence d'au moins 0,3%, et plus préférablement au moins 0,5%, par rapport à la masse totale de la composition. D'autre part, la teneur massique en gomme xanthane dans la composition selon la présente invention peut être d'au plus 1,2%, de préférence au plus 0,7%, par rapport à la masse totale de la composition.

De préférence, la teneur massique en gomme xanthane dans la composition selon la présente invention est de 0,2% à 1,2%, de préférence de 0,5% à 0,7%, par rapport à la masse totale de la composition. Cela permet de combiner une meilleure stabilité et sensorialité de la composition tout en évitant les phénomènes de cristallisation dus aux agents lipophiles tels que les beurres ou les alcools gras.

La composition cosmétique selon la présente invention comprend de la gomme de gellane.

La gomme de gellane est un polysaccharide naturel linéaire comprenant des résidus glucose, rhamnose et acide glucuronique. Les résidus acides glucuroniques sont responsables de la nature anionique de la gomme gellane et peuvent être neutralisés dans les produits commerciaux avec des cations. Les gommes gellane sont des exopolysaccharides produits par la fermentation de la bactérie *Sphingomonas elodea.* A titre d'exemples non limitatifs de gommes gellane commercialement disponibles, on peut citer les composés de no. CAS 71010-52-1 ou les produits commercialisés sous les dénominations KELCOGEL CG-HA de la société CP KELCO, Nutrier GL-L30C ou GL-H40C de la société SIDERE.

La teneur massique en gomme gellane dans la composition selon la présente invention peut être d'au moins 0,05%, de préférence d'au moins 0,1%, et plus préférablement au moins 0,2%, par rapport à la masse totale de la composition. D'autre part, la teneur massique en gomme gellane dans la composition selon la présente invention peut être d'au plus 0,5%, de préférence au plus 0,3%, par rapport à la masse totale de la composition.

De préférence, la teneur massique en gomme gellane dans la composition selon la présente invention est de 0,05% à 0,5%, de préférence de 0,1% à 0,3%, par rapport à la masse totale de la composition. Cela permet de combiner une meilleure stabilité et sensorialité de la composition tout en évitant les phénomènes de cristallisation.

De préférence, la somme des teneurs massiques en gomme xanthane et en gomme gellane dans la composition selon la présente invention est d'au moins 0,3%, de préférence d'au moins 0,5%, par rapport à la masse totale de la composition.

De préférence, la somme des teneurs massiques en gomme xanthane et en gomme gellane dans la composition selon la présente invention est d'au plus 1,7%, de préférence au plus 1,2%, par rapport à la masse totale de la composition.

De préférence, la somme des teneurs massiques en gomme xanthane et en gomme gellane dans la composition selon la présente invention est de 0,3% à 1,7%, de préférence de 0,5% à 1,2%, par rapport à la masse totale de la composition.

Cela permet de combiner une meilleure stabilité et sensorialité de la composition tout en évitant les phénomènes de cristallisation.

### Phase grasse :

La composition selon l'invention comprend au moins une phase grasse dispersée dans une phase aqueuse. En d'autres termes, la composition selon l'invention comprend une phase grasse discontinue. La phase grasse est organique et non-miscible dans l'eau.

La phase grasse (également appelée ici phase huileuse) comprend au moins un corps gras.

Par "corps gras", on entend au sens de l'invention un composé organique non miscible dans l'eau à température ambiante (25°C) et à pression atmosphérique (1,013 . 10⁵ Pa ; 760 mm de Hg). Plus particulièrement, la solubilité dans l'eau de ces composés est inférieure à 5%, de préférence à 1% et plus préférentiellement inférieure à 0,1% en masse. Ce type de composé présente généralement dans sa structure une chaine hydrocarbonée comportant au moins 6 atomes de carbone.

Les corps gras peuvent être liquides ou non liquides à température ambiante (25°C) et à pression atmosphérique (1,013 . 10⁵ Pa ; 760 mm de Hg).

Dans la composition selon l'invention, la phase grasse comprend au moins une huile. La phase grasse peut être essentiellement constituée d'une huile ou d'un mélange de plusieurs huiles.

Par "huile" on entend un "corps gras" qui est liquide à température ambiante (25°C), et à pression atmosphérique (101 325 Pa ; 760 mm de Hg). Les huiles ou corps gras liquides peuvent être volatiles ou non volatiles.

Par "huile volatile", on entend une huile susceptible de s'évaporer au contact de la peau en moins d'une heure, à température ambiante et à pression atmosphérique. L'huile volatile est liquide à température ambiante et peut avoir une pression de vapeur allant de 0,13 Pa à 40 000 Pa (de 10⁻³ à 300 mm Hg).

Par "huile non volatile", on entend une huile restant sur la peau à température ambiante et pression atmosphérique au moins plusieurs heures. Elles ont notamment une pression de vapeur inférieure à 0,13 Pa (inférieure à 10⁻³ mm de Hg).

Dans le cadre de la présente invention, on utilise de préférence des corps gras hydrocarbonés, en particulier d'origine naturelle.

Avantageusement, la composition selon l'invention est exempte de corps gras siliconés et de corps gras fluorés.

On entend par "hydrocarboné", au sens de la présente invention, un composé contenant au moins un groupement hydrocarboné (c'est-à-dire un groupement contenant des atomes d'hydrogène et de carbone), et éventuellement des atomes d'oxygène, d'azote, de soufre et/ou de phosphore. Le composé hydrocarboné peut par exemple contenir des groupes alcool, ester, éther, acide carboxylique, amine et/ou amide. Plus particulièrement, dans le cadre de la présente invention, un composé hydrocarboné ne contient pas d'atome de silicium ou de fluor.

On entend par "siliconé", au sens de la présente invention, un composé comprenant au moins un atome de silicium, et notamment au moins un groupe Si-O. On peut citer par exemple les organopolysiloxanes.

On entend par "fluoré" un composé contenant au moins un atome de fluor.

Comme corps gras utilisables dans le cadre de la présente invention, on peut par exemple citer les hydrocarbures comprenant 6 à 16 atomes de carbone, les huiles hydrocarbonées comprenant plus de 16 atomes de carbone, les huiles non siliconées, les huiles végétales (triglycérides, acides gras), les glycérides, les alcools gras, les esters d'acide gras et/ou d'alcool gras différents des triglycérides, les éthers d'acide gras et leurs mélanges.

Les hydrocarbures en C6 à C16 sont de préférence d'origine végétale. Ils peuvent être linéaires ou ramifiés, et sont de préférence des alcanes. A titre d'exemple, on peut citer le n-décane (C10), le n-undécane (C11), le n-dodécane (C12), le n-tridécane (C13), le n-tétradécane (C14), le n-pentadécane (C15), le n-hexadécane (C16), ou un mélange d'alcanes (par exemple de undécane et tridécane).

Les huiles hydrocarbonées (ou hydrocarbures comprenant plus de 16 atomes de carbone) sont de préférence d'origine végétale. Il peut s'agir de composés linéaires ou ramifiés. A titre d'exemple d'huiles hydrocarbonées, on peut citer le squalane. Les huiles végétales sont de préférence choisies parmi les triglycérides, tels que les triglycérides d'acides caprylique/caprique. Des exemples non limitatifs d'huiles végétales incluent l'huile d'amande douce, l'huile de macadamia, l'huile de noisette, l'huile d'arachide, l'huile de sésame, l'huile de noix, l'huile d'argan, l'huile de jojoba, l'huile de calendula, l'huile d'abricot, l'huile de tournesol, l'huile d'olive, l'huile de maïs, l'huile de soja, l'huile de chanvre, l'huile de colza, l'huile de coton, l'huile de coprah, l'huile de graine de courge, l'huile de pépins de raisin, l'huile d'arara, l'huile de ricin, l'huile d'avocat, l'huile de mirabelle, l'huile de beurre de karité et leurs mélanges.

Les glycérides sont des esters de glycérol et d'un (monoglycérides) ou plusieurs (diglycérides et triglycérides) acides gras. Ils sont de préférence d'origine naturelle. On peut citer par exemple les triglycérides des acides caprique/caprylique.

Les alcools gras convenant à la mise en œuvre de l'invention peuvent être plus particulièrement choisis parmi les alcools saturés ou insaturés, linéaires ou ramifiés, comportant de 6 à 30 atomes de carbone. Des exemples non limitatifs incluent l'alcool cétylique, l'alcool cétéarylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique. Dans le cadre de la présente invention, les alcools gras utilisables comme corps gras sont de préférence d'origine naturelle.

Les esters dits "gras" présentent au moins un groupement hydrocarboné, linéaire ou ramifié, saturé ou insaturé, comprenant au moins 6 atomes de carbone, de préférence de 8 à 30 atomes de carbone, éventuellement substitué, en particulier par un ou plusieurs groupements hydroxyle. Ils peuvent être choisis parmi les esters d'acide gras et/ou d'alcools gras, éventuellement hydroxylés. Ces esters, différents des triglycérides mentionnés auparavant, peuvent être notamment choisis parmi les esters de mono ou polyacides aliphatiques saturés ou insaturés, linéaires ou ramifiés en C1 à C26 et de mono ou polyalcools aliphatiques saturés ou insaturés, linéaires ou ramifiés en C1 à C26, le nombre total de carbone des esters étant supérieur ou égal à 6. Des exemples non limitatifs d'esters d'acide gras et/ou d'alcools gras incluent le béhénate d'octyldodécyle, le béhénate d'isocétyle, le lactate de linoléyle, le laurate d'isocétyle, laurate d'hexyle, le stéarate d'isocétyle, l'oléate d'isodécyle, l'isononanoate d'isononyle, l'isononanoate d'octyle, le ricinoléate de méthyle acétyle, le palmitate d'isostéaryle, le palmitate d'éthyle, d'isopropyle, le palmitate de 2-éthyl-hexyle, les myristates d'alkyles tels que le myristate d'isopropyle, de butyle, de cétyle, le stéarate de butyle, le stéarate d'isobutyle, le stéarate ou l'isostéarate d'isopropyle, l'isostéarate d'isostéaryle, le stéarate d'octyle, l'hydroxystéarate d'octyle, le lactacte d'isostéaryle, l'adipate de diisopropyle, le malate de dioctyle, le laurate de 2- hexyldécyle, le caprate/caprylate d'ethyl2-hexyle (ou caprate/caprylate d'octyle), le néopentanoate d'isostéaryle, d'isodécyle, les heptanoates (tels que l'heptanoate d'isostéaryle), les octanoates (tels que l'octanoate de cétyle, l'octanoate de cetearyl, l'octanoate de tridécyle).

Les éthers d'acide gras présentent au moins un groupement hydrocarboné, linéaire ou ramifié, saturé ou insaturé, comprenant au moins 6 atomes de carbone, de préférence de 8 à 30 atomes de carbone, éventuellement substitué, en particulier par un ou plusieurs groupements hydroxyle. Ils peuvent être plus particulièrement choisis parmi les éthers comportant de 6 à 24 atomes de carbone. Ils sont de préférence d'origine végétale, comme par exemple le dicaprylyl éther.

De préférence, la phase grasse comprend au moins un corps gras non liquide à température ambiante (25°C) et à pression atmosphérique (101 325 Pa; 760 mm de Hg).

Par corps gras non liquide, on entend un composé solide ou un composé présentant une viscosité supérieure à 2 Pa.s à température de 25°C et à un taux de cisaillement de 1 s⁻¹, mesurée avec un Rhéomat RM 180 (généralement au mobile 1 ou 2).

Parmi les corps gras non liquides, on peut par exemple citer les beurres, les cires et de façon plus globale les alcools gras non liquides (tels que l'alcool myristique, l'alcool cétylique, l'alcool stéarylique, etc.), les esters d'acide gras et/ou d'alcool gras non liquides (tels que le béhénate d'octyldodécyle, le béhénate d'isocétyle, le lactate de cétyle, l'octanoate de stéaryle, l'octanoate d'octyle, etc.), les amines et éthers gras non liquides.

Avantageusement, la composition selon l'invention comprend au moins un beurre d'origine végétale. La présence d'un beurre végétal apporte généralement des propriétés émollientes mais aussi sensorielles. Cela permet notamment de donner de la consistance à la composition. Un inconvénient est que leur présence peut conduire à un aspect grumeleux à cause de phénomènes de cristallisation. Or les inventeurs ont mis en évidence que l'association particulière selon l'invention permet d'éviter de façon surprenante ces phénomènes de cristallisation.

Par "beurre" (ou "corps gras pâteux"), on entend au sens de la présente invention un corps gras (donc lipophile) à changement d'état solide/liquide réversible et comportant une fraction liquide et une fraction solide à la température de 25°C et à pression atmosphérique (760 mm Hg). En d'autres termes, la température de fusion commençante du composé pâteux peut être inférieure à 25°C. La fraction liquide du composé pâteux (beurre) mesurée à 25°C peut par exemple représenter 10 à 90 % en masse du composé.

De préférence, le ou les beurres présentent une température de fin de fusion inférieure à 60°C.

Au sens de la présente invention, la température de fin de fusion correspond à la température à laquelle 95% de l'échantillon a fondu. La mesure de la température de fusion et la détermination de la température de fin de fusion peuvent être effectuées selon le protocole décrit dans le document WO2020/127383.

Avantageusement, la teneur massique en beurre dans la composition selon l'invention est de 2% à 10%, de préférence de 5 à 8%, par rapport à la masse totale de la composition.

Avantageusement, la composition selon l'invention comprend au moins un agent de viscosant de phase grasse choisi parmi les alcools gras, de préférence d'origine naturelle.

Par "agent viscosant de phase grasse", on entend au sens de la présente invention un composé lipophile qui, de par sa présence, permet de conférer de la consistance à la composition, c'est-à-dire d'augmenter la viscosité de la composition.

De préférence, l'agent de consistance est un alcool gras comprenant 12 à 22 atomes de carbone. A titre d'exemples non limitatifs d'alcools gras utilisables, on peut citer l'alcool myristique ou myristylique, l'alcool cétylique, l'alcool stéarylique ou l'alcool oléique. La présence d'alcool gras comme agent viscosant, en particulier solide ou cireux à température ambiante (25°C), permet de conférer de la consistance à la composition selon l'invention (à la prise et à l'application). De façon surprenante, l'association particulière selon l'invention permet d'éviter l'effet savon à l'application qui est généralement observé avec les alcools gras, et ceci sans nécessiter l'introduction d'huile de silicone.

Avantageusement, la teneur massique en alcool gras comme agent viscosant dans la composition selon l'invention est de 2% à 6%, de préférence de 3% à 5%, par rapport à la masse totale de la composition. Cela est préférable pour la sensorialité de la composition.

La composition selon l'invention peut avoir une teneur massique totale en corps gras d'au moins 5%, de préférence au moins 8%, par rapport à la masse totale de la composition.

La composition selon l'invention peut avoir une teneur massique totale en corps gras d'au plus 25%, de préférence au plus 23%, par rapport à la masse totale de la composition.

De préférence, la composition selon l'invention peut avoir une teneur massique totale en corps gras de 5% à 25%, de préférence de 8% à 23%, par rapport à la masse totale de la composition. Cela est préférable pour la stabilité et la sensorialité de la composition.

En outre, la composition selon l'invention peut avoir une teneur massique totale en huile d'au moins 5%, de préférence au moins 8%, par rapport à la masse totale de la composition.

La composition selon l'invention peut avoir une teneur massique totale en huile d'au plus 15%, de préférence au plus 10%, par rapport à la masse totale de la composition. De préférence, la composition selon l'invention peut avoir une teneur massique totale en huile de 5% à 15%, de préférence de 8% à 10%, par rapport à la masse totale de la composition. Cela est préférable pour la stabilité et la sensorialité de la composition. La phase grasse peut comprendre en outre des ingrédients additionnels solubles ou miscibles dans l'huile comme par exemple des solvants, des agents de formulation et/ou des actifs liposolubles.

Selon un mode préféré de l'invention, la composition selon l'invention est exempte de composé siliconé.

### Phase aqueuse :

La composition selon l'invention comprend une phase aqueuse continue.

La phase aqueuse comprend de l'eau. Elle peut comprendre en outre des ingrédients additionnels solubles ou miscibles dans l'eau comme par exemple des solvants hydrosolubles, des agents de formulation hydrosolubles et/ou des actifs hydrosolubles.

Parmi les solvants solubles ou miscibles dans l'eau, on peut citer par exemple les monoalcools à chaîne courte par exemple en C1 à C4 (comme l'éthanol, l'isopropanol), les polyols, linéaires ou ramifiés, par exemple comprenant 3 à 8 atomes de carbone, par exemple comprenant 2 à 6 fonctions -OH (comme l'éthylène glycol, le 1,2-propylène glycol, le 1,3-butylène glycol, l'hexylèneglycol, le diéthylèneglycol, le dipropylène glycol, le 2-éthoxyéthanol, le diéthylène glycol monométhyl éther, le triéthylène glycol monométhyl éther, le glycérol, et le sorbitol), et leurs mélanges.

De préférence, la composition selon l'invention a une teneur massique en eau d'au moins 50%, de préférence au moins 55%, par rapport à la masse totale de la composition. Cela permet d'obtenir de meilleures propriétés sensorielles et de stabilité de la composition.

La composition selon l'invention peut avoir une teneur massique en eau d'au plus 90%, de préférence au plus 80%, de préférence au plus 70%, par rapport à la masse totale de la composition. Cela est préférable pour la faisabilité et stabilité de la composition.

### Composés additionnels :

La composition selon l'invention peut comprendre en outre un ou plusieurs agents de formulation, notamment des actifs ou des excipients cosmétiques classiques.

Ces agents de formulation peuvent être choisis de manière appropriée en fonction de l'utilisation de la composition de l'invention.

Des exemples non limitatifs d'agents de formulation incluent les actifs, hydrophiles ou lipophiles, les solvants organiques (par exemple les alcools et polyols inférieurs), les agents rhéologiques (épaississants ou gélifiants) ioniques ou non ioniques, hydrophiles ou lipophiles, les adoucissants, les humectants, les opacifiants, les stabilisants, les émollients, les agents anti-mousse, les agents filmogènes, les émulsifiants, les tensioactifs (anioniques, cationiques, non ioniques ou amphotères), les charges (par exemple les poudres matifiantes, les poudres de toucher actives soft focus, etc.), les filtres UV (ou agents photoprotecteurs, organiques et/ou inorganiques, actif dans l'UVA et/ou l'UVB), les colorants, les agents chélateurs (chélatant), les agents alcalinisants ou acidifiants, des ajusteurs de pH, les conservateurs, les parfums ou tout autre ingrédient habituellement utilisé dans le domaine cosmétique et/ou dermatologique. Les quantités de ces différents composés sont celles classiquement utilisées dans les domaines considérés. Ces composés, selon leur nature, peuvent être introduits dans la phase grasse ou dans la phase aqueuse.

La composition selon l'invention peut comprendre au moins un actif. Parmi les actifs, on peut citer par exemple :
- les agents anti-pollution et/ou agent anti-radicalaire et/ou agents antioxydants (coenzyme Q10 ou ubiquinone) ;
- les agents éclaircissants, les agents dépigmentants, les agents pro-pigmentants et/ou les agents autobronzants ;
- les agents anti-âge ;
- les agents tenseurs ou antirides, tels que les protéines végétales et leurs hydrolysats (par exemple l'extrait de protéines de soja) ;
- les agents hydratants (tels que les polyols comme par exemple la glycérine) ;
- les agents anti-inflammatoires (tels que par exemple l'acide glycyrrhétinique et ses sels) ;
- les agents apaisants (allantoïne, bisabolol, eau de bleuet),
- les agents anti-microbiens ou antibactériens (tels que l'acide salicylique) ;
- les agents tenseurs ;
- les agents raffermissants ;
- les vitamines (telles que le rétinol ou vitamine A, l'acide ascorbique ou vitamine C, le tocophérol ou vitamine E, la niacinamide ou vitamine PP ou B3, le panthénol ou vitamine B5 la biotine ou vitamine B8 et leurs dérivés tels que par exemple les esters de ces vitamines) ;
- les huiles essentielles.

Grâce à ses connaissances en matière de cosmétique, l'homme du métier est à même de choisir les agents de formulation à ajouter aux compositions de l'invention et leurs quantités en fonction des propriétés recherchées sans altérer substantiellement les effets liés à la composition selon l'invention.

La composition selon l'invention peut avoir différents profils rhéologiques et se présenter sous la forme d'une crème, d'un gel, d'un baume.

De préférence la composition selon l'invention a une texture épaisse et se présente sous la forme d'un baume.

En particulier, la composition peut avoir une viscosité, à 20° C et pression atmosphérique (1,013. 10⁵ Pa), allant de 20 000 à 80 000 Pa.s., en particulier de 40 000 à 65 000 Pa.s. mesurée avec le Mobile 6 Vitesse 6 Brookfield RV sur Rheomat.

Avantageusement, la composition selon l'invention présente, par rapport aux compositions similaires disponibles dans le commerce, une naturalité élevée voire supérieure, pouvant être de plus de 99% d'ingrédients d'origine naturelle, tout en présentant à la fois une sensorialité et une stabilité comparable aux compositions formulées avec des émulsionnants et/ou des épaississants d'origine synthétique. Les inventeurs ont notamment démontré que la combinaison particulière d'ingrédients permet d'obtenir une texture lisse non grumeleuse, non collante, non freinante et qui savonne et peluche peu à l'application.

De façon avantageuse, la composition selon l'invention permet également de lisser la peau et d'améliorer l'hydratation, la douceur, l'apparence et l'éclat de la peau. Ainsi, l'invention a également pour objet l'utilisation de la composition cosmétique telle que définie ci-dessus comme produit de soin, de préférence pour hydrater, protéger, traiter la peau et/ou atténuer les signes de vieillissement cutané.

La présente invention a également pour objet un procédé cosmétique de soin de la peau comprenant l'application sur la peau d'une composition telle que définie ci-avant.

### Exemples

L'invention est illustrée plus en détail par les exemples non limitatifs présentés ci-après. Les composés ou matières premières utilisés sont nommés par leur nom INCI. En l'absence d'indication contraire, les pourcentages indiqués sont des pourcentages massiques. La mention "qsp" signifie que la quantité doit être suffisante pour que la composition atteigne 100% de sa masse finale.

### Exemple 1 :

Les compositions 1 à 5 sont préparées selon le mode opératoire décrit ci-après avec les proportions en % indiquées dans le tableau 1 ci-dessous. Les différentes compositions sont également évaluées au niveau de leur aspect, de leur stabilité dans le temps et de leurs propriétés d'application.

**Tableau 1.**

| **P** | **NOMS INCI** | **Compositions (%)** | Hors invention | | | | Invention |
|---|---|---|---|---|---|---|---|
| | | | **1** | **2** | **3** | **4** | **5** |
| A | AQUA/WATER/EAU | | 58,87 | 58,87 | 58,87 | 58,87 | 58,87 |
| | SODIUM PHYTATE^{(a)} | | 0,1 | 0,1 | 0,1 | 0,1 | 0,1 |
| | CITRIC ACID^{(h)} | | 0,05 | 0,05 | 0,05 | 0,05 | 0,05 |
| B | PROPANEDIOL⁽ⁱ⁾ | | 3 | 3 | 3 | 3 | 3 |
| | ETHYLHEXYLGLYCERIN^{(b)} | | 0,2 | 0,2 | 0,2 | 0,2 | 0,2 |
| C | GLYCERIN⁽ⁱ⁾ | | 3 | 3 | 3 | 3 | 3 |
| | BUTYLENE GLYCOL^{(k)} | | 2 | 2 | 2 | 2 | 2 |
| | XANTHAN GUM (and) AQUA/WATER/EAU^{(c)} | | 1 | 0,7 | 0,7 | 0,7 | 0,7 |
| | GELLAN GUM^{(d)} | | - | - | 0,3 | 0,3 | 0,3 |
| | ALGIN^{(e)} | | - | 0,3 | - | - | - |
| D | SORBITAN STEARATE (and) SUCROSE COCOATE^{(f)} | | 5 | 5 | 5 | - | 3 |
| E | POLYGLYCERYL-6 STEARATE (and) POLYGLYCERYL-6 BEHENATE^{(g)} | | - | - | - | 5 | 2 |
| | SQUALANE^{(l)} | | 8 | 8 | 8 | 8 | 8 |
| | COCOS NUCIFERA (COCONUT) OIL^{(m)} | | 3 | 3 | 3 | 3 | 3 |
| | BUTYROSPERMUM PARKII (SHEA) BUTTER⁽ⁿ⁾ | | 5 | 5 | 5 | 5 | 5 |
| | CETYL ALCOHOL^{(o)} | | 5 | 5 | 5 | 5 | 5 |
| F | ALCOHOL^{(p)} | | 5 | 5 | 5 | 5 | 5 |
| | ETHYLENE BRASSYLATE^{(q)} | | 0,01 | 0,01 | 0,01 | 0,01 | 0,01 |
| G | AQUA/WATER/EAU | | 0,72 | 0,72 | 0,72 | 0,72 | 0,72 |
| | CITRIC ACID^{(h)} | | 0,05 | 0,05 | 0,05 | 0,05 | 0,05 |
| **Total :** | | | **100** | **100** | **100** | **100** | **100** |
| **% d'ingrédients d'origine naturelle** | | | **99.69** | **99.69** | **99.69** | **99.69** | **99.69** |

Dans le tableau ci-dessus :
- la colonne de gauche intitulée "P" indique les phases dans lesquelles les composés sont présents ;
- les références entre parenthèses (à côté des noms INCI des composés) indiquent les noms commerciaux des matières premières utilisées comprenant ces composés. Le détail de ces matières premières est donné ci-dessous :
   ^{(a)} DERMOFEEL PA-12 (de nom INCI : sodium phytate) commercialisé par la société EVONIK Nutrition & Care GmbH.
   ^{(b)} SENSIVA SC 50 (ingrédient d'origine synthétique, de nom INCI : ethylhexylglycerin) commercialisé par la société SCHÜLKE FRANCE SARL.
   ^{(c)} RHODICARE XC (de nom INCI : xanthan gum (and) aqua/water/eau) commercialisé par la société DANISCO.
   ^{(d)} KELCOGEL CG-HA (de nom INCI : gellan gum) commercialisé par la société CP KELCO.
   ^{(e)} SATIALGINE VCG 171 (de nom INCI : Algin) commercialisé par la société UNIPEX.
   ^{(f)} ARLACEL 2121 (de nom INCI : sorbitan stearate (and) sucrose cocoate) commercialisé par la société CRODA.
   ^{(g)} TEGO CARE PBS 6 (de nom INCI : polyglyceryl-6 stearate (and) polyglyceryl-6 behenate) commercialisé par la société EVONIK Nutrition & Care GmbH.
   ^{(h)} Acide citrique monohydraté en poudre, commercialisé par la société MERCK
   ⁽ⁱ⁾ ZEMEA Propanediol, commercialisé par la société DuPont Tate & Lyle Bio Products.
   ^{(j)} Wilfarin EP997, commercialisé par la société WILMAR.
   ^{(k)} BRONTIDE BG, commercialisé par la société AZELIS
   ^{(l)} Squalane Neossance ou Squalane Biossance de la société AMYRIS
   ^{(m)} HYDROBASE 24/26 (nom INCI: Cocos nucifera oil), commercialisé par la société LABORATOIRES PROD'HYG (France)
   ⁽ⁿ⁾ Cetiol SB 45 (nom INCI : Butyrospermum parkii), commercialisé par la société BASF
   ^{(o)} Alcool Cetylique 98%, commercialisé par la société STEARINERIE DUBOIS
   ^{(p)} Alcool Surfin 96,2, commercialisé par la société TEREOS
   ^{(q)} MUSK T, commercialisé par la société TAKASAGO

### Mode opératoire :

Une phase A est préparée en solubilisant dans un bécher le phytate de sodium puis l'acide citrique dans l'eau sous agitation Rayneri équipé d'une défloculeuse jusqu'à dissolution du phytate de sodium et de l'acide citrique.

Une phase B est préalablement préparée en mélangeant le propanediol et l'éthylhexylglycérine. La phase B est ensuite ajoutée à la phase A et le tout est agité 5 minutes à 300 rpm (rotation par minute).

Une phase C est préparée en mélangeant la glycérine et le butylène glycol puis en prédispersant, dans cette phase, le ou les épaississants aqueux polysaccharidiques (la gomme xanthane et, le cas échéant, la gomme gellane ou l'algin). Puis la phase C est ajoutée au mélange aqueux précédent sous agitation Rayneri équipé d'une défloculeuse. L'agitation est maintenue jusqu'au déploiement des polysaccharides (environ 20 min à 1000 rpm). Le mélange, sous agitation, est chauffé à 55°C à l'aide d'une plaque chauffante.

La phase D (mélange d'émulsionnants ester de sucre et ester de sorbitan) est ensuite ajoutée au mélange aqueux précédent, toujours à 55°C. Puis le tout est agité 15 min sous agitation avec défloculeuse à 75°C.

Une phase E est préparée en mélangeant les esters de polyglycérol avec les corps gras (le squalane, l'huile de coco, le beurre de karité et l'alcool gras). Cette phase E est ensuite chauffée à 75°C.

L'émulsion est ensuite réalisée en ajoutant, sous agitation défloculeuse Rayneri, la phase E dans le mélange obtenu (phases A, B, C et D). L'agitation est maintenue à 1200 rpm pendant 15 minutes entre 75°C et 70°C. Une émulsion lisse, brillante, blanche et épaisse qui tient à la spatule est obtenue.

Le mélange est ensuite refroidi à 30°C avant d'ajouter la phase F (mélange d'alcool et de brassylate d'éthylène) sous agitation défloculeuse Rayneri. Le mélange obtenu est agité 10 min puis refroidi à 28°C (le pH est alors d'environ 5,9)

Le pH est ensuite ajusté en ajoutant au mélange précédent la phase G (acide citrique dans l'eau) sous agitation défloculeuse (le pH est alors proche de 5).

Des émulsions huile-dans-eau sont ainsi obtenues.

### Méthodes d'évaluation :

### Mesure de la viscosité :

La viscosité est mesurée après obtention de la composition (émulsion H/E), après 24 heures de stockage de la composition en étuve à 20°C.

La viscosité est mesurée à l'aide d'un viscosimètre Brooksfield, modèle RVT (Brookfield Engineering Laboratories, Inc.), à une température ambiante d'environ 20°C. Cet appareil mesure le couple nécessaire pour faire tourner, à une vitesse « V » donnée, un mobile « M » de taille donnée (sélectionnée conformément aux recommandations de fonctionnement standard du fabricant), en immersion dans le fluide à tester. Ici la viscosité est mesurée avec le mobile no. RV S06 à une vitesse de 6 rpm (rotation par minute). Les mesures sont exprimées en cP (centipoise ou Pa.s). Les résultats sont répertoriés dans le tableau 2 ci-après.

### Evaluation de la stabilité :

La stabilité des compositions ci-dessus a été étudiée après préparation et/ou après stockage dans des conditions accélérées à 50°C pendant 1 mois (dans une étuve thermostatée).

Les compositions ont été évaluées par analyse sensorielle (aspect, couleur et odeur). L'aspect global et la couleur sont évalués à l'œil nu et au microscope (Microscope Olympus BH2 Grossissement 10x10) afin de contrôler la couleur, la surface, les éventuels phénomènes de relargage, de séparation de phases, de sédimentation ou d'agglomération, ainsi que la présence éventuelle de précipités, de grains, déphasage ou volute. L'odeur est comparée avec deux compositions témoins (l'une conservée dans l'obscurité à 20°C et l'autre conservée à 4°C au réfrigérateur).

Les résultats sont répertoriés dans le tableau 2 ci-dessous. Les compositions 1 à 5 ont une viscosité plus ou moins élevée. L'ensemble des compositions a un aspect initial lisse sauf la composition 3 qui a un aspect très légèrement grumeleux à l'état initial.

Les études de stabilité après stockage dans les conditions accélérées montrent que les compositions 1 et 2, qui ne comprennent pas l'association des deux épaississants gomme xanthane et gomme gellane, ne sont pas stables, soit parce que l'émulsion casse, soit parce qu'un phénomène de cristallisation se produit avec apparition de grains. Quant à la composition 3 qui ne comprend pas d'ester de polyglycérol, la stabilité de l'émulsion est acceptable mais son aspect devient légèrement grumeleux et cireux après stockage à 50°C. Contrairement aux compositions 1 à 3, la composition 5 selon l'invention est stable, blanche et lisse et garde une texture crème épaisse (texture baume, de viscosité supérieure à 40 000 Pa.s).

### Evaluation des propriétés sensorielles :

Les compositions 3 à 5, de stabilité satisfaisante, ont un aspect initial lisse avec une texture douce, non collante et non « pâteuse », avec toutefois un aspect légèrement grumeleux pour la composition 3. Les propriétés sensorielles de ces compositions sont évaluées sur un panel de six personnes expertes : l'effet savon, l'effet peluche et l'effet freinant sont évalués selon les modes opératoires ci-après. Pour chacun de ces modes opératoires, les compositions sont déposées à l'aide d'une pipette sur la peau préalablement nettoyée à l'eau et au savon liquide et essuyée à l'aide d'un mouchoir en papier. Les résultats sont répertoriés dans le tableau 2 ci-après.

### Evaluation de l'effet savon :

Pour chaque composition, 0,5 mL de produit est appliqué sur l'avant-bras. On réalise des mouvements circulaires avec la main (1 tour par seconde) jusqu'à pénétration du produit. On relève le moment où le produit savonne le plus et on évalue visuellement ce potentiel savon sur une échelle de 0 (pas d'effet savon) à 4 (zone d'application blanche qui recouvre quasiment toute la peau). Dans le tableau 2, la moyenne des 6 panelistes experts est retenue.

Les résultats montrent que la composition 4, qui ne contient pas l'association d'émulsionnants ester de sucre et ester de sorbitan, présente un effet savon très important, contrairement à ce que l'on observe lors de l'application de la composition 5 selon l'invention qui présente un faible effet de savonnage.

### Evaluation de l'effet peluche :

Pour chaque composition, 0,5 mL de produit est appliqué sur l'avant-bras. On réalise 15 mouvements circulaires avec la main (15 tours en 15 secondes), puis on attend 15 secondes, et on réalise à nouveau 15 mouvements circulaires en 15 secondes, puis on laisse reposer 1 minute et on réalise à nouveau 15 mouvements circulaires en 15 secondes. On relève le moment où le produit forme le plus de peluches et on évalue ce potentiel de peluchage sur une échelle de 0 (pas d'effet peluche) à 4 (présence très forte de peluches). Dans le tableau 2, la moyenne des 6 panelistes experts est retenue.

Les résultats montrent que la composition 3, qui ne contient pas d'ester de polyglycérol, peluche fortement lors de l'application sur la peau, contrairement à la composition 5 selon l'invention.

### Evaluation de l'effet freinant ou glissant :

Pour chaque composition, 0,5 mL de produit est appliqué sur l'avant-bras. On réalise 15 mouvements circulaires avec la main (15 tours en 15 secondes). On évalue le « glissant » à l'application, c'est-à-dire la facilité d'application du produit et sa capacité à recouvrir une zone déterminée, par opposition à l'effet freinant, c'est-à-dire au potentiel du produit à s'accrocher à la peau pendant l'application. L'effet freinant est évalué sur une échelle de 0 (pas d'effet freinant, bonne glisse) à 4 (le produit s'accroche fortement à la peau pendant l'application). Dans le tableau 2, la moyenne des 6 panelistes experts est retenue.

La composition 5 selon l'invention est très facile d'application avec une bonne glisse, tandis que les compositions comparatives 3 et 4 sont plus freinantes et moins faciles à appliquer.

### Résultats :

Les résultats sont répertoriés dans le tableau 2 ci-dessous.

**Tableau 2.**

| **Compositions** | Hors invention | | | | Invention |
|---|---|---|---|---|---|
| | **1** | **2** | **3** | **4** | **5** |
| **Viscosité en cP (à J=24h)** | 33167 | 20500 | 35667 | 54667 | 48667 |
| **Aspect (à J=24h)** | lisse | lisse | Légèrement grumeleux | lisse | lisse |
| **Stabilité et Aspect (après stockage à 50°C pendant 1 mois)** | Casse totale de l'émulsion après 15 jours à 50°C | Apparition de grains après 15 jours à 50°C | Emulsion stable, aspect grumeleux et cireux | Emulsion stable, aspect lisse | Emulsion stable, aspect lisse |
| **Effet savon** | NA | NA | 1 | 4 | 2 |
| **Effet freinant** | NA | NA | 2 | 2 | 1 |
| **Effet peluche** | NA | NA | 4 | 1 | 1 |

Dans le tableau ci-dessus, la mention "NA" signifie que l'effet n'a pas été évalué (Non Applicable).

En conclusion, la composition 5 selon l'invention comprenant l'association particulière d'épaississants et émulsionnants, permet de concilier l'ensemble des propriétés recherchées telles que la stabilité, la consistance et la sensorialité avec un effet savon et un effet peluche peu présents et une application facile avec une bonne glisse du produit sur la peau. En outre, la composition 5 selon l'invention a aspect lisse, onctueux et non grumeleux et permet d'éviter les phénomènes de cristallisation. Enfin, la composition 5 selon l'invention présente une texture douce, non collante, non grasse au toucher et légère, qui ne procure pas de sensation de lourdeur.

La combinaison particulière d'ingrédients selon l'invention permet ainsi d'obtenir une composition avec un taux de naturalité élevé, tout en présentant les propriétés sensorielles et de stabilité requises.

### Exemple 2 :

Un autre exemple de composition selon l'invention est donné avec la composition 6 comprenant les proportions en % indiquées dans le tableau 3 ci-dessous.

**Tableau 3.**

| **P** | **NOMS INCI** | **Composition (%)** | Invention |
|---|---|---|---|
| | | | **6** |
| A | AQUA/WATER/EAU | | 63,48 |
| | SODIUM PHYTATE^{(a)} | | 0,1 |
| | CITRIC ACID^{(h)} | | 0,05 |
| B | PROPANEDIOL⁽ⁱ⁾ | | 8 |
| C | GLYCERIN^{(j)} | | 3 |
| | XANTHAN GUM (and) AQUA/WATER/EAU^{(c)} | | 0,7 |
| | GELLAN GUM^{(d)} | | 0,3 |
| D | SORBITAN STEARATE (and) SUCROSE COCOATE^{(f)} | | 3 |
| E | POLYGLYCERYL-6 STEARATE (and) POLYGLYCERYL-6 BEHENATE^{(g)} | | 2 |
| | SQUALANE^{(l)} | | 8 |
| | COCOS NUCIFERA (COCONUT) OIL^{(m)} | | 1 |
| | BUTYROSPERMUM PARKII (SHEA) BUTTER⁽ⁿ⁾ | | 2 |
| | CETYL ALCOHOL^{(o)} | | 2 |
| F | PHENETHYL ALCOHOL | | 0,5 |
| | Parfum | | 0,1 |
| G | ALCOHOL^{(p)} | | 5 |
| H | AQUA/WATER/EAU | | 0,72 |
| | CITRIC ACID^{(h)} | | 0,05 |
| **Total :** | | | **100** |
| % **d'ingrédients d'origine naturelle** | | | **99,5** |

La composition 6 est préparée selon le mode opératoire décrit précédemment pour le mélange des phases A à E (obtention d'une émulsion lisse).

Le mélange est ensuite refroidi à 30°C avant d'ajouter la phase F (contenant l'alcool phényléthylique) sous agitation défloculeuse Rayneri. Le mélange obtenu est agité 10 min.

La phase G (alcool) est ensuite ajoutée sous agitation défloculeuse Rayneri.

Le pH est ensuite ajusté en ajoutant au mélange précédent la phase H (acide citrique dans l'eau) sous agitation défloculeuse (le pH est alors proche de 5). Une émulsion huile-dans-eau blanche, brillante et semi-épaisse est ainsi obtenue.

La composition 6 est également évaluée au niveau de son aspect, de sa stabilité dans le temps et de ses propriétés d'application (effet savon, effet freinant et effet peluche).

La composition 6 selon l'invention est suffisamment consistante pour être conditionnée en pot. Cette composition présente un pH (à J=24h) de 5,09 et une viscosité (à J= 24h) de 38667 cP.

La composition 6 présente également un aspect lisse, onctueux et non grumeleux qui est stable dans le temps, sans phénomène de recristallisation. Sa consistance est également préservée dans le temps.

De plus, la composition 6 combine les propriétés sensorielles recherchées, comparables à celles de la composition 5, avec un effet savon et un effet peluche peu présents et une application facile avec une bonne glisse du produit sur la peau. Enfin, la composition 6 présente une texture douce et non collante, avec un fondant légèrement moins gras que celui de la composition 5 selon l'invention.

En conclusion, l'association d'ingrédients selon la présente invention permet d'obtenir des compositions huile-dans-eau naturelles, stables et homogènes dans le temps, tout en combinant à la fois une application facile avec une bonne glisse et une sensorialité tout à fait satisfaisante en limitant l'effet savon et l'effet peluche.

## Revendications

1. Composition cosmétique sous forme d'émulsion huile-dans-eau comprenant au moins une phase grasse dispersée dans une phase aqueuse et comprenant :
a) au moins un ester de sucre et d'acide gras ;
b) au moins un ester d'acide gras du sorbitan ;
c) au moins un ester de polyglycérol et d'acide gras ;
d) au moins une gomme xanthane ; et
e) au moins une gomme gellane.

2. Composition cosmétique selon la revendication 1, dans laquelle l'ester de sucre et d'acide gras est le sucrose cocoate.

3. Composition cosmétique selon la revendication 1 ou 2, dans laquelle l'ester d'acide gras du sorbitan est le sorbitan stéarate.

4. Composition cosmétique selon l'une quelconque des revendications précédentes, comprenant au moins deux esters de polyglycérol et d'acide gras.

5. Composition cosmétique selon l'une quelconque des revendications précédentes, dans laquelle dans laquelle le au moins un ester de polyglycérol et d'acide gras comprend 6 motifs glycérol.

6. Composition cosmétique selon l'une quelconque des revendications précédentes, dans laquelle le au moins un ester de polyglycérol et d'acide gras est choisi parmi le polyglycéryl-6 stéarate, le polyglycéryl-6 béhénate et leur mélange.

7. Composition cosmétique selon l'une quelconque des revendications précédentes, dans laquelle la teneur massique en ester de polyglycérol et d'acide gras est d'au moins 0,5%, par rapport à la masse totale de la composition.

8. Composition cosmétique selon l'une quelconque des revendications précédentes, dans laquelle la somme des teneurs massiques en gomme xanthane et en gomme gellane est d'au moins 0,3%, de préférence d'au moins 0,5%, par rapport à la masse totale de la composition.

9. Composition cosmétique selon l'une quelconque des revendications précédentes, la composition comprenant en outre un beurre d'origine végétale.

10. Composition cosmétique selon l'une quelconque des revendications précédentes, dans laquelle la phase grasse comprend au moins un agent viscosant de phase grasse choisi parmi les alcools gras.

11. Composition cosmétique selon l'une quelconque des revendications précédentes, la composition étant exempte de composé siliconé.

12. Composition cosmétique selon l'une quelconque des revendications précédentes, la composition étant exempte d'épaississant synthétique aqueux.

13. Utilisation non-thérapeutique cosmétique d'une composition selon l'une quelconque des revendications précédentes pour le soin cosmétique de la peau, de préférence pour hydrater, protéger, traiter la peau et/ou atténuer les signes de vieillissement cutané.

## Patentansprüche

1. Kosmetische Zusammensetzung in Form einer Öl-in-Wasser-Emulsion, umfassend mindestens eine in einer wässrigen Phase dispergierte Fettphase und enthaltend:
a) mindestens einen Ester aus Zucker und Fettsäure;
b) mindestens einen Fettsäureester des Sorbitans;
c) mindestens einen Ester aus Polyglyzerin und Fettsäure;
d) mindestens ein Xanthan-Gummi; und
e) mindestens ein Gellan-Gummi.

2. Zusammensetzung nach Anspruch 1, bei der der Ester aus Zucker und Fettsäure Saccharosecocoat ist.

3. Kosmetische Zusammensetzung nach Anspruch 1 oder 2, **dadurch gekennzeichnet, dass** der Fettsäureester des Sorbitans Sorbitanstearat ist.

4. Kosmetische Zusammensetzung nach einem der vorhergehenden Ansprüche, enthaltend mindestens zwei Ester aus Polyglyzerin und Fettsäure.

5. Kosmetische Zusammensetzung nach einem der vorhergehenden Ansprüche, wobei der mindestens ein Ester aus Polyglyzerin und Fettsäure 6 Glycerineinheiten umfasst.

6. Kosmetische Zusammensetzung nach einem der vorhergehenden Ansprüche, wobei der mindestens ein Ester aus Polyglyzerin und Fettsäure ausgewählt ist aus Polyglyceryl-6-Stearat, Polyglyceryl-6-Behenat und deren Mischung.

7. Kosmetische Zusammensetzung nach einem der vorhergehenden Ansprüche, in der der Gehalt an Estern aus Polyglyzerin und Fettsäure, bezogen auf die Gesamtmasse der Zusammensetzung, mindestens 0,5 % beträgt.

8. Kosmetische Zusammensetzung nach einem der vorhergehenden Ansprüche, bei der die Summe der Massengehalte an Xanthan-gummi und Gellan-gummi mindestens 0,3 %, vorzugsweise mindestens 0,5 %, bezogen auf die Gesamtmasse der Zusammensetzung, beträgt.

9. Kosmetische Zusammensetzung nach einem der vorhergehenden Ansprüche, wobei die Zusammensetzung außerdem eine Butter pflanzlichen Ursprungs enthält.

10. Kosmetische Zusammensetzung nach einem der vorhergehenden Ansprüche, **dadurch gekennzeichnet, dass** die Fettphase mindestens einen Fettphasenviskosmacher enthält, der unter den Fettalkoholen ausgewählt ist.

11. Kosmetische Zusammensetzung nach einem der vorhergehenden Ansprüche, wobei die Zusammensetzung frei von Silikonverbindungen ist.

12. Kosmetische Zusammensetzung nach einem der vorhergehenden Ansprüche, wobei die Zusammensetzung frei von wässrigen synthetischen Verdickungsmitteln ist.

13. Nichttherapeutische kosmetische Verwendung einer Zusammensetzung nach einem der vorhergehenden Ansprüche zur kosmetischen Hautpflege, vorzugsweise zur Befeuchtung, zum Schutz, zur Behandlung der Haut und/oder zur Abschwächung der Zeichen der Hautalterung.

## Claims

1. Composition cosmetic in the form of an oil-in-water emulsion comprising at least one fatty phase dispersed in an aqueous phase and comprising :
a) at least one fatty acid sugar ester ;
b) at least one sorbitan fatty acid ester;
c) at least one polyglycerol fatty acid ester;
d) at least one xanthan gum; and
e) at least one gellan gum.

2. Cosmetic composition according to claim 1, in which the sugar fatty acid ester is sucrose cocoate.

3. Cosmetic composition according to claim 1 or 2, in which the sorbitan fatty acid ester is sorbitan stearate.

4. Cosmetic composition according to any one of the preceding claims, comprising at least two polyglycerol fatty acid esters.

5. Cosmetic composition according to any one of the preceding claims, in which the at least one polyglycerol fatty acid ester comprises 6 glycerol units.

6. Cosmetic composition according to any one of the preceding claims, in which the at least one polyglycerol fatty acid ester is chosen from 6-polyglyceryl stearate, 6-polyglyceryl behenate and mixtures thereof.

7. Cosmetic composition according to any one of the preceding claims, in which the content by mass of polyglycerol fatty acid ester is at least 0.5% relative to the total mass of the composition.

8. Cosmetic composition according to any one of the preceding claims, in which the sum of the mass contents of xanthan gum and gellan gum is at least 0.3%, preferably at least 0.5%, relative to the total mass of the composition.

9. Cosmetic composition according to any one of the preceding claims, the composition further comprising a butter of plant origin.

10. Cosmetic composition according to any one of the preceding claims, in which the fatty phase comprises at least one fatty phase viscosifier chosen from fatty alcohols.

11. Cosmetic composition according to any one of the preceding claims, the composition being free of silicone compound.

12. Cosmetic composition according to any one of the preceding claims, the composition being free of aqueous synthetic thickener.

13. Non-therapeutic cosmetic use of a composition according to any one of the preceding claims for cosmetic skin care, preferably for moisturising, protecting, treating the skin and/or attenuating the signs of skin ageing.
